# EUROPEAN PATENT SPECIFICATION

(11) **EP 3 252 043 B1**
(45) Date of publication and mention of the grant of the patent: **30.09.2020**
(21) Application number: 16743545.2
(22) Date of filing: 29.01.2016
(51) Int. Cl.: C07D 295/088, A61K 9/127, A61K 31/7088, A61K 45/00, A61K 47/22, A61K 48/00, A61P 43/00, C07D 311/00

(54) **CATIONIC LIPID**
KATIONISCHES LIPID
LIPIDE CATIONIQUE

(30) Priority: 30.01.2015 JP 2015016786
(43) Date of publication of application: 06.12.2017
(73) Proprietor: NOF Corporation, Shibuya-ku Tokyo 150-6019 (JP); National University Corporation Hokkaido University, Sapporo-shi, Hokkaido 060-0808 (JP)
(72) Inventor: NAKAI, Yuta, Kawasaki-shi Kanagawa 210-0865 (JP); TANGE, Kota, Kawasaki-shi Kanagawa 210-0865 (JP); AKITA, Hidetaka, Sapporo-shi Hokkaido 060-0808 (JP); HARASHIMA, Hideyoshi, Sapporo-shi Hokkaido 060-0808 (JP); TOGASHI, Ryohei, Sapporo-shi Hokkaido 060-0808 (JP); MIURA, Naoya, Sapporo-shi Hokkaido 060-0808 (JP); MAETA, Mio, Sapporo-shi Hokkaido 060-0808 (JP)
(74) Representative: J A Kemp LLP
(86) International application number: PCT/JP2016/052690
(87) International publication number: WO 2016/121942

(56) References cited:
- WO-A1-2013/073480
- JP-A- 2005 508 394
- JP-A- 2011 121 966
- JP-A- 2011 509 074
- JP-A- 2012 509 272
- JP-A- 2012 526 134
- US-A- 6 153 434
- US-A1- 2014 335 157
- KEDIKA,B. ET AL.: 'Synthesis and Gene Transfer Activities of Novel Serum Compatible Reducible Tocopherol-Based Cationic Lipids' MOLECULAR PHARMACEUTICS vol. 9, no. 5, 01 January 2012, pages 1146 - 1162, XP055470382 DOI: 10.1021/MP200435Y
- MARKOV,O.O. ET AL.: 'Novel cationic liposomes provide highly efficient delivery of DNA and RNA into dendritic cell progenitors and their immature offsets' JOURNAL OF CONTROLLED RELEASE vol. 160, no. 2, 01 January 2012, pages 200 - 210, XP028926641 DOI: 10.1016/J.JCONREL.2011.11.034
- TANG,F. ET AL.: 'Use of Dithiodiglycolic Acid as a Tether for Cationic Lipids Decreases the Cytotoxicity and Increases Transgene Expression of Plasmid DNA in Vitro' BIOCONJUGATE CHEMISTRY vol. 10, no. 5, 01 January 1999, pages 791 - 796, XP009084473 DOI: 10.1021/BC990016I
- ZHANG,Y. ET AL.: 'Cationic gemini lipids with cyclen headgroups: interaction with DNA and gene delivery abilities' RSC ADVANCES vol. 4, no. 83, 01 January 2014, pages 44261 - 44268, XP055470447 DOI: 10.1039/C4RA05974C

## Description

### [Technical Field]

The present invention relates to a cationic lipid having improved nucleic acid delivery efficiency, a lipid membrane structure containing same, and use thereof.

### [Background Art]

Nucleic acid treatment is a therapeutic method for suppressing expression of a pathogenic protein by delivering the nucleic acid (RNA) into the cytoplasm, and gene therapy is a therapeutic method for promoting expression of a protein useful for the treatment by delivering the nucleic acid (DNA) into the nucleus. In these treatment methods, delivery of the nucleic acid into cells is important. However, delivery of nucleic acid into cells is difficult, since nucleic acid is rapidly degraded when used alone by enzymes in the blood. Therefore, practicalization of these therapeutic drugs requires a carrier for delivery of the nucleic acid into the cells.

In view of the property of the carrier that delivers foreign substances into cells, it is necessary to exhibit a large effect with a small amount of use. That is, the nucleic acid delivery carrier is required to increase the delivery amount of the nucleic acid per unit carrier incorporated into the cytoplasm, that is, to increase the nucleic acid delivery efficiency into the cytoplasm.

Virus vectors represented by retrovirus and adenovirus are carriers with high nucleic acid delivery efficiency. On the other hand, they are associated with problems such as formation of tumor caused by insertion of the viral vector into the genome and nonspecific influence on cells other than the target cells. In view of these, the development of non-viral carriers is ongoing. Of those, a nucleic acid delivery carrier using a cationic lipid (lipid membrane structure) is a non-viral carrier used most generally.

To increase nucleic acid delivery efficiency with a nucleic acid delivery carrier using cationic lipid, pharmacokinetics (e.g., stability in blood, accumulation property in target cells such as tumor and the like, and the like) need to be improved. Furthermore, to increase delivery efficiency of nucleic acid into the cytoplasm, improvement of intracellular dynamics (e.g., uptake into cells, escape from endosome, release of nucleic acid from carrier in the cytoplasm and the like), besides the aforementioned pharmacokinetics, also becomes necessary (non-patent document 1).

Cationic lipids are roughly composed of a hydrophobic moiety and a hydrophilic moiety. As its constitution, the hydrophobic moiety comprises a hydrophobic group such as fatty acid group, sterol group and the like, and the hydrophilic moiety comprises a cationic group such as amino group, and the like. As the composition of the cationic lipid, many structures comprising two hydrophobic groups per one hydrophilic group (hereinafter to be referred to as "two-chain cationic lipid") are known.

As mentioned above, a nucleic acid delivery carrier using a cationic lipid requires improvement of pharmacokinetics and intracellular dynamics. Since nucleic acid and cellular membrane are anionic, the cationic group of a cationic lipid, which electrostatically interacts with them, has been found to play an important role in solving these problems. Therefore, among cationic lipids, cationic groups, namely, amino groups, are being developed mainly.

For improvement of intracellular dynamics, a method using a cationic lipid having a quaternary amine is known. For example, known two-chain cationic lipid 1,2-Dioleoyl-3-dimethylammonium propane (hereinafter to be referred to as "DOTAP") having a quaternary amine can form a positively-charged lipid membrane structure by an electrostatic interaction between an amino group of DOTAP and an anionic nucleic acid. The positively-charged lipid membrane structure interacts with an anionic cellular membrane to increase uptake into the cell. However, since the electrostatic interaction between DOTAP having a quaternary amine and nucleic acid is too strong, release of the nucleic acid from the carrier is problematically difficult (non-patent document 2).

On the other hand, various studies have also been made on tertiary amine. As a known two-chain cationic lipid having tertiary amine, 1,2-Dioleoyl-3-dimethylamino propane (hereinafter to be referred to as "DODAP") can be mentioned. It is described that DODAP can form a lipid membrane structure by electrostatic interaction with nucleic acid, and becomes a carrier capable of delivering nucleic acid to the target cell (non-patent document 2).

Non-patent document 3 describes pharmacokinetics. In this document, pKa of two-chain cationic lipid is adjusted to near neutral. It is shown that a lipid membrane structure using the cationic lipid is stable in blood for a long time after intravenous injection, and accumulated in the tumor site.

Non-patent document 4 describes intracellular dynamics. This document describes as regards two-chain type cationic lipids that pKa as a lipid membrane structure can be adjusted to a value advantageous for intracellular endosomes escape, by changing the structure around the amino group. It is stated that this promotes endosomal escape and clearly improves nucleic acid delivery efficiency.

In addition, cationic lipids having hydrophobic group and tertiary amino groups with different amino group number have also been developed. For example, patent documents 1 and 3 describe cationic lipids having a structure in which compounds having one hydrophobic group and one hydrophilic group are linked with each other by a biodegradable disulfide bond. The documents show that the cationic lipid can improve pharmacokinetics such as stability in blood, tumor targeting property and the like. In addition, it has been clarified that the cationic lipid can improve intracellular dynamics such as increase in the delivery efficiency of nucleic acid into the cytoplasm and the like, since it exhibits higher nucleic acid delivery efficiency as compared to known cationic lipids such as DOTAP and DODAP.

However, despite the technical progress in this field, the nucleic acid delivery efficiency into the cytoplasm, which is achieved by a lipid membrane structure using cationic lipid, is not fully satisfactory.

As described in patent document 2, it is useful to contain many amino groups when intracellular deliverability is to be improved. However, when many amino groups are contained, release of nucleic acid from the carrier in the cell is suppressed. Therefore, improvement of nucleic acid delivery efficiency cannot be expected.

### [Document List]

### [Patent documents]

patent document 1: WO 2013/073480
patent document 2: JP-A-2011-121966
patent document 3: US20140335157

### [non-patent documents]

non-patent document 1: Molecular Therapy 13 (4): 786-794, 2006
non-patent document 2: Biomaterials 29 (24-25): 3477-96, 2008
non-patent document 3: Journal of Controlled Release 107: 276-287, 2005
non-patent document 4: Nature Biotechnology 28: 172-176, 2010

### [SUMMARY OF THE INVENTION]

### [Problems to be Solved by the Invention]

An object of the present invention is to provide a cationic lipid useable as a nucleic acid delivery carrier, a lipid membrane structure using a cationic lipid, and a nucleic acid-introducing agent using a cationic lipid.

In addition, the present invention aims to provide a method of achieving nucleic acid introduction by using a nucleic acid-introducing agent containing a cationic lipid.

### [Means of Solving the Problems]

To improve nucleic acid delivery efficiency into the cytoplasm, it is necessary to increase the number of amino groups, improve intracellular deliverability, and efficiently release nucleic acid in the cytoplasm.

The present inventor took note of this technical problem and conducted intensive studies to find that a cationic lipid having a structure in which compounds composed of a hydrophobic group and a hydrophilic group in which piperazine is a tertiary amine are linked to each other by a disulfide bond (structure having 2 hydrophobic groups and 4 hydrophilic groups, hereinafter to be also referred to as the cationic lipid of the present invention) has high nucleic acid delivery efficiency, which resulted in the completion of the present invention.

Therefore, the present invention encompasses the following.
[1] A cationic lipid represented by the formula (1) 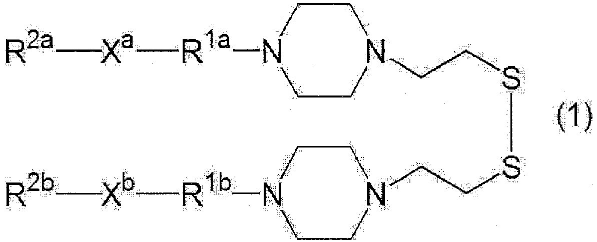
   in the formula (1), R^{1a} and R^{1b} are each independently an alkylene group or oxydialkylene group having not more than 8 carbon atoms,
   X^{a} and X^{b} are each independently an ester bond, an amide bond, a carbamate bond, or an ether bond, and
   R^{2a} and R^{2b} are each independently a sterol residue, a liposoluble vitamin residue, or an aliphatic hydrocarbon group having 13 - 23 carbon atoms.
[2] The cationic lipid of [1], wherein R^{1a} and R^{1b} are each independently an alkylene group.
[3] The cationic lipid of [1] or [2], wherein X^{a} and X^{b} are ester bonds.
[4] The cationic lipid of any of [1] - [3], wherein R^{2a} and R^{2b} are each independently a liposoluble vitamin residue, or an aliphatic hydrocarbon group having 13 - 23 carbon atoms.
[5] The cationic lipid of any of [1] - [4], wherein R^{2a} and R^{2b} are each independently a liposoluble vitamin residue.
[6] The cationic lipid of any of [1] - [4], wherein R^{2a} and R^{2b} are each independently an aliphatic hydrocarbon group having 13 - 23 carbon atoms.
[7] A lipid membrane structure comprising the cationic lipid of any of [1] to [6] as a membrane-constituting lipid.
[8] A nucleic acid-introducing agent, which comprises the cationic lipid of any of [1] to [6] or the lipid membrane structure of [7].
[9] A nucleic acid-introducing agent, which is the cationic lipid of any of [1] - [6], or the lipid membrane structure of [7], encapsulating an anti-inflammatory agent.
[10] A method of delivering a nucleic acid into a cell, comprising contacting the nucleic acid-introducing agent of [8] or [9], which encapsulates the nucleic acid, with the cell in vitro.
[11] A nucleic acid-introducing agent of [8] or [9], which encapsulates the nucleic acid, for use in a method of therapy which comprises introducing the nucleic acid into a target cell of a living organism.

### [Effect of the Invention]

The present invention relates to a cationic lipid. The cationic lipid can form a lipid membrane structure, and can form a nucleic acid-introducing agent containing the cationic lipid. Since a lipid membrane structure containing the cationic lipid can have pKa near neutral, it is stable in blood and accumulates in tumor. In addition, the disulfide bond contained in the cationic lipid of the present invention is cleaved in the intracellular reductive environment, and release of the encapsulated substance (nucleic acid) is promoted. Thus, a nucleic acid-introducing agent using the cationic lipid of the present invention can achieve high nucleic acid delivery efficiency of the nucleic acid to be delivered into the cytoplasm.

In addition, when a nucleic acid is introduced using the cationic lipid or lipid membrane structure of the present invention, degradation of the nucleic acid by serum components can be suppressed. Thus, it is advantageous for nucleic acid introduction in the presence of serum or nucleic acid introduction in vivo.

### [Brief Description of the Drawings]

Fig. 1 shows gene expression activity of various MENDs (multifunctional envelope-type nano device) prepared from various cationic lipids (Myr-C3M, TS-C3M, TS-PZ4C2).
Fig. 2 shows time-course changes in the gene expression activity of MEND prepared from TS-PZ4C2 having an adjusted amount of encapsulated Dex-Pal.
Fig. 3 shows gene expression activity in the liver after intravenous administration of pDNA, or a transgene agent prepared from a commercially available transfection reagent, which encapsulates pDNA, and MEND prepared from TS-PZ4C2, which encapsulates pDNA.
Fig. 4 shows gene expression activity in the liver after intravenous administration of MEND prepared from TS-C3M or MEND prepared from TS-PZ4C2, which encapsulates pDNA.
Fig. 5, the upper panel, provides photographs showing accumulation after intravenous administration of various MENDs prepared from various cationic lipids (Myr-C3M, TS-PZ4C2, L-PZ4C2, O-PZ4C2), which carry liposoluble fluorescence dye, in each organ and tumor. The middle panel shows the amount of accumulation in the liver 24 hr after the administration. The lower panel shows the amount of accumulation in tumor 24 hr after the administration.
Fig. 6 shows gene expression activity in tumor 48 hr after intravenous administration of various MENDs prepared from various cationic lipids (Myr-C3M, L-PZ4C2, O-PZ4C2) encapsulating pDNA.
Fig. 7 shows an antitumor effect after intravenous administration of various MENDs prepared from L-PZ4C2 encapsulating a gene.

### [Description of Embodiments]

While the embodiments of the present invention are explained in the following, the present invention is not limited thereto.

The present invention provides a compound represented by the formula (1) (hereinafter to be also referred to as the compound of the present invention, or the cationic lipid of the present invention).

R^{1a} and R^{1b} are each independently an alkylene group or oxydialkylene group having not more than 8 carbon atoms, preferably an alkylene group having not more than 8 carbon atoms.

The alkylene group having not more than 8 carbon atoms may be linear or branched, preferably linear. The number of carbons contained in the alkylene group is preferably not more than 6, most preferably not more than 4. Specific examples of the alkylene group having not more than 8 carbon atoms include methylene group, ethylene group, propylene group, isopropylene group, tetramethylene group, isobutylene group, pentamethylene group, hexamethylene group, heptamethylene group, octamethylene group and the like, preferably methylene group, ethylene group, propylene group and tetramethylene group, most preferably ethylene group.

The oxydialkylene group having not more than 8 carbon atoms is an alkylene group via an ether bond (alkylene-O-alkylene), and the total of the carbon number of the two alkylene groups is not more than 8. Here, the two alkylenes may be the same or different, preferably the same. Specific examples of the oxydialkylene group having not more than 8 carbon atoms include oxydimethylene group, oxydiethylene group, oxydipropylene group, oxydibutylene group and the like. Preferred are oxydimethylene group, oxydiethylene group, and oxydipropylene group, and most preferred is oxydiethylene group.

R^{1a} may be the same as or different from R^{1b}, and R^{1a} is preferably the same group as R^{1b}.

X^{a} and X^{b} are each independently an ester bond, an amide bond, a carbamate bond, or an ether bond, preferably an ester bond or an amide bond, most preferably an ester bond. While the binding direction of X^{a} and X^{b} is not limited, when X^{a} and X^{b} are ester bonds, a structure having R^{2a}-CO-O-R^{1a}- or R^{2b}-CO-OR^{1b}- is preferable.

X^{a} may be the same as or different from X^{b}, and X^{a} is preferably the same group as X^{b}.

R^{2a} and R^{2b} are each independently a sterol residue, a liposoluble vitamin residue or an aliphatic hydrocarbon group having 13 - 23 carbon atoms, preferably a liposoluble vitamin residue or an aliphatic hydrocarbon group having 13 - 23 carbon atoms, most preferably an aliphatic hydrocarbon group. From the aspect of organ (particularly liver) specificity, R^{2a} and R^{2b} are also preferably liposoluble vitamin residues.

As the "sterol residue", sterol excluding a reactive functional group (e.g., hydroxyl group) involved in the binding with X^{a} or X^{b}, or a residue derived from a sterol derivative can be mentioned, and preferred is a residue derived from a sterol derivative. The sterol derivative is, for example, a sterol hemiester obtained by reacting a hydroxyl group of sterol with one of the carboxylic acids of dicarboxylic acid (in this case, the other carboxylic acid becomes a reactive functional group). Examples of the sterol include cholesterol, cholestanol, stigmasterol, β-sitosterol, lanosterol, ergosterol and the like, with preference given to cholesterol and cholestanol. Examples of the dicarboxylic acid include malonic acid, succinic acid, glutaric acid, adipic acid and the like, with preference given to succinic acid or glutaric acid. Specific examples of the sterol derivative include cholesterol hemisuccinic acid ester, cholesterol hemiglutaric acid ester and the like.

As the "liposoluble vitamin residue", a liposoluble vitamin excluding a reactive functional group (e.g., hydroxyl group) involved in the binding with X^{a} or X^{b}, or a residue derived from a liposoluble vitamin derivative can be mentioned, and preferred is a residue derived from a liposoluble vitamin derivative. The liposoluble vitamin derivative is, for example, a liposoluble vitamin hemiester obtained by reacting a hydroxyl group of liposoluble vitamin with one of the carboxylic acids of dicarboxylic acid (in this case, the other carboxylic acid becomes a reactive functional group). Examples of the liposoluble vitamin include retinoic acid, retinol, retinal, ergosterol, 7-dehydrocholesterol, calciferol, cholecalciferol, dihydroergocalciferol, dihydrotachysterol, tocopherol, tocotrienol and the like. Preferable examples thereof are retinoic acid and tocopherol, which is most preferably tocopherol. Examples of the dicarboxylic acid include malonic acid, succinic acid, glutaric acid, adipic acid and the like, with preference given to succinic acid and glutaric acid. Specific examples of the liposoluble vitamin derivative include tocopherol hemisuccinic acid ester, tocopherol hemiglutaric acid ester and the like.

The aliphatic hydrocarbon group having 13 - 23 carbon atoms may be linear or branched, preferably linear. The aliphatic hydrocarbon group may be saturated or unsaturated. In the case of an unsaturated hydrocarbon group, the aliphatic hydrocarbon group contains 1 - 6, preferably 1 - 3, most preferably 1 - 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 13 - 21, most preferably 13 - 17, when it is a straight chain. Examples of the aliphatic hydrocarbon group having 13 - 23 carbon atoms include tridecyl group, tetradecyl group, pentadecyl group, hexadecyl group, heptadecyl group, octadecyl group, nonadecyl group, icosyl group, henicosyl group, docosyl group, tricosyl group, tridecenyl group, tetradecenyl group, pentadecenyl group, hexadecenyl group, heptadecenyl group, octadecenyl group, nonadecenyl group, icosenyl group, henicosenyl group, docosenyl group, tricosenyl group, tridecadienyl group, tetradecadienyl group, pentadecadienyl group, hexadecadienyl group, heptadecadienyl group, octadecadienyl group, nonadecadienyl group, icosadienyl group, henicosadienyl group, docosadienyl group, octadecatrienyl group, icosatrienyl group, icosatetraenyl group, icosapentaenyl group, docosahexaenyl group, methyldodecyl group, methyltridecyl group, methyltetradecyl group, methylpentadecyl group, methylheptadecyl group, methyloctadecyl group, methylnonadecyl group, methylicosyl group, methylhenicosyl group, methyldocosyl group, ethylundecyl group, ethyldodecyl group, ethyltridecyl group, ethyltetradecyl group, ethylpentadecyl group, ethylheptadecyl group, ethyloctadecyl group, ethylnonadecyl group, ethylicosyl group, ethylhenicosylgroup, hexylheptyl group, hexylnonyl group, heptyloctyl group, heptyldecyl group, octylnonyl group, octylundecyl group, nonyldecyl group, decylundecyl group, undecyldodecyl group, hexamethylundecyl group and the like. As the straight chain, preferred are tridecyl group, pentadecyl group, heptadecyl group, nonadecyl group, henicosylgroup, heptadecenyl group, heptadecadienyl group, particularly preferably, tridecyl group, heptadecyl group, heptadecenyl group, and heptadecadienyl group. As the branched one, preferred are methylpentadecyl group, hexylnonyl group, heptyldecyl group, octylundecyl group, and hexamethylundecyl group, and particularly preferred are methylpentadecyl group, hexylnonyl group, and heptyldecyl group.

In one embodiment, an aliphatic hydrocarbon group having 13 - 23 carbon atoms, which is derived from fatty acid, aliphatic alcohol, or aliphatic amine, is used. When R^{2a} is derived from fatty acid, X^{a} is an ester bond or an amide bond, and aliphatic series-derived carbonyl carbon is included in X^{a}. When R^{2b} is derived from fatty acid, X^{b} is an ester bond or an amide bond, and aliphatic series-derived carbonyl carbon is included in X^{b}. Specific examples of the aliphatic hydrocarbon group include heptadecadienyl group when linoleic acid is used as fatty acid, and heptadecenyl group when oleic acid is used as fatty acid.

R^{2a} may be the same as or different from R^{2b}, and R^{2a} is preferably the same group as R^{2b}.

In one embodiment, R^{1a} is the same as R^{1b}, X^{a} is the same as X^{b}, and R^{2a} is the same as R^{2b}.

In one embodiment,
R^{1a} and R^{1b} are each independently an alkylene group having not more than 8 carbon atoms (1 - 8 carbon atoms),
X^{a} and X^{b} are each an ester bond, and
R^{2a} and R^{2b} are each independently a liposoluble vitamin residue (e.g., group derived from tocopherol hemisuccinic acid ester).

In one embodiment,
R^{1a} and R^{1b} are each independently an alkylene group having not more than 8 carbon atoms (1 - 8 carbon atoms),
X^{a} and X^{b} are each an ester bond, and
R^{2a} and R^{2b} are each independently an aliphatic hydrocarbon group having 13 - 23 carbon atoms (e.g., heptadecadienyl group, heptadecenyl group).

In one embodiment,
R^{1a} and R^{1b} are each independently an alkylene group having not more than 8 carbon atoms (1 - 8 carbon atoms),
X^{a} and X^{b} are each an ester bond,
R^{2a} and R^{2b} are each a liposoluble vitamin residue (e.g., a group derived from tocopherol hemisuccinic acid ester),
R^{1a} is the same as R^{1b}, and
R^{2a} is the same as R^{2b}.

In one embodiment,
R^{1a} and R^{1b} are each independently an alkylene group having not more than 8 carbon atoms (1 - 8 carbon atoms),
X^{a} and X^{b} are each an ester bond,
R^{2a} and R^{2b} are each an aliphatic hydrocarbon group having 13 - 23 carbon atoms (e.g., heptadecadienyl group, heptadecenyl group),
R^{1a} is the same as R^{1b} and
R^{2a} is the same as R^{2b}.

In one embodiment,
R^{1a} and R^{1b} are each an ethylene group,
X^{a} and X^{b} are each -CO-O-, and
R^{2a} and R^{2b} are each independently a liposoluble vitamin residue (e.g., a group derived from tocopherol hemisuccinic acid ester).

In one embodiment,
R^{1a} and R^{1b} are each an ethylene group,
X^{a} and X^{b} are each -CO-O-, and
R^{2a} and R^{2b} are each independently an aliphatic hydrocarbon group having 13 - 23 carbon atoms (e.g., heptadecadienyl group, heptadecenyl group).

In one embodiment,
R^{1a} and R^{1b} are each an ethylene group,
X^{a} and X^{b} are each -CO-O-,

R^{2a} and R^{2b} are each a liposoluble vitamin residue (e.g., a group derived from tocopherol hemisuccinic acid ester), and
R^{2a} is the same as R^{2b}.

In one embodiment,
R^{1a} and R^{1b} are each an ethylene group,
X^{a} and X^{b} are each -CO-O-,
R^{2a} and R^{2b} are each an aliphatic hydrocarbon group having 13 - 23 carbon atoms (e.g., heptadecadienyl group, heptadecenyl group), and
R^{2a} is the same as R^{2b}.

Specific examples of the cationic lipid of the present invention include the following TS-PZ4C2, L-PZ4C2, and O-PZ4C2.

**Table 1**

| name of cationic lipid | structure |
|---|---|
| TS-PZ4C2 | 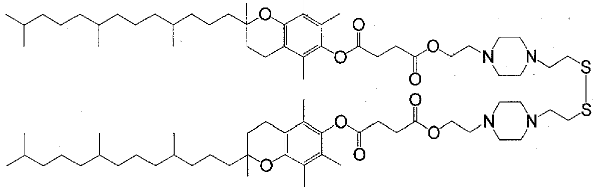 |
| L-PZ4C2 | 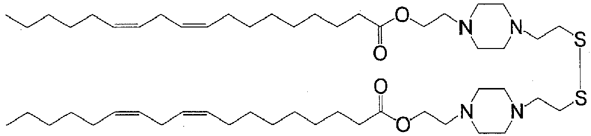 |
| O-PZ4C2 | 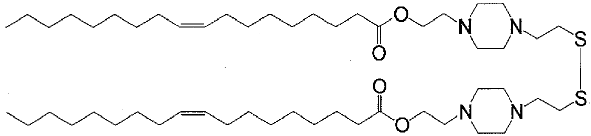 |

The production method of the compound of the present invention is explained now.

The compound of the present invention has an -S-S-(disulfide) bond. Therefore, the production method includes, for example, a method including producing SH (thiol) compound having R^{2a}-X^{a}-R^{1a}- and SH (thiol) compound having R^{2b}-X^{b}-R^{1b}-, and subjecting them to oxidation (coupling) to give the compound of the present invention containing -S-S- bond, a method including sequentially synthesizing necessary parts to a compound containing an -S-S- bond to finally obtain the compound of the present invention and the like. Preferred is the latter method.

While a specific example of the latter method is shown below, production methods are not limited to these.

Examples of the starting compound include two terminal carboxylic acid, two terminal amine, two terminal isocyanate, two terminal alcohol, two terminal alcohol having a leaving group such as methanesulfonyl group and the like, a two terminal carbonate having a leaving group such as p-nitrophenylcarbonate group and the like, and the like, which contain -S-S- bond.

For example, when a compound wherein R^{1a} and R^{1b} are each an ethylene group, X^{a} and X^{b} are the same and X (ester bond, amide bond, carbamate bond, or ether bond), and R^{2a} and R^{2b} are the same and R² (sterol residue, liposoluble vitamin residue, or aliphatic hydrocarbon group having 13 - 23 carbon atoms) is produced, both terminal functional groups of compound (I) containing an -S-S- bond are reacted with a secondary amino group at the 1-position of a piperazine derivative having a functional group at the 4-position via an ethylene group (hereinafter to be referred to as "compound (II)"), and the functional group in the derivative (II) is reacted with a functional group in compound (III) containing R²-X, whereby the compound of the present invention containing an -S-S- bond, two piperazine skeletons, R^{1a} and R^{1b}, X^{1a} and X^{1b}, and R^{2a} and R^{2b} can be obtained.

In the reaction of compound (I) and compound (II), a base catalyst such as potassium carbonate, sodium carbonate, potassium hydroxide and the like may be used as a catalyst, or the reaction may be performed without a catalyst. Preferably, potassium carbonate or sodium carbonate is used as a catalyst.

The amount of catalyst is 0.1 - 100 molar equivalents, preferably, 0.1 - 20 molar equivalents, more preferably 0.1 - 5 molar equivalents, relative to compound (I). The amount of compound (II) to be charged is 1 - 50 molar equivalents, preferably 1 - 10 molar equivalents, relative to compound (I).

The solvent to be used for the reaction of compound (I) and compound (II) is not particularly limited as long as it is a solvent or aqueous solution that does not inhibit the reaction. For example, ethyl acetate, dichloromethane, chloroform, acetonitrile, toluene and the like can be mentioned. Among these, toluene, chloroform and acetonitrile are preferable.

The reaction temperature is -20 to 150°C, preferably 0 to 80°C, more preferably 20 to 50°C, and the reaction time is 1 - 48 hr, preferably 2 - 24 hr.

When the reaction product of compound (I) and compound (II) (hereinafter to be referred to as reaction product (I)) is reacted with compound (III), an alkali catalyst such as potassium carbonate, sodium carbonate, potassium hydroxide and the like, or an acid catalyst such as p-toluenesulfonic acid, methanesulfonic acid and the like may be used, like the catalyst used for the reaction of compound (I) and compound (II), or the reaction may be performed without a catalyst.

Reaction product (I) and compound (III) may be directly reacted using condensing agents such as dicyclohexylcarbodiimide (hereinafter to be referred to as "DCC"), diisopropylcarbodiimide (hereinafter to be referred to as "DIC"), 1-ethyl-3-(3-dimethylaminopropyl)carbodiimide hydrochloride (hereinafter to be referred to as "EDC") and the like, or compound (III) may be converted to anhydride and the like by using a condensing agent and then reacted with the reaction product (I).

The amount of compound (III) to be charged is 1 - 50 molar equivalents, preferably 1 - 10 molar equivalents, relative to the reaction product (I).

The catalyst to be used for reacting reaction product (I) with compound (III) is appropriately selected according to the functional groups to be reacted.

The amount of catalyst is 0.05 - 100 molar equivalents, preferably 0.1 - 20 molar equivalents, more preferably 0.2 - 5 molar equivalent, relative to the reaction product (I).

The solvent to be used for the reaction of the reaction product (I) and compound (III) is not particularly limited as long as it is a solvent or aqueous solution that does not inhibit the reaction. For example, ethyl acetate, dichloromethane, chloroform, acetonitrile, toluene and the like can be mentioned. Among these, chloroform and toluene are preferable.

The reaction temperature is 0 to 150°C, preferably 0 to 80°C, more preferably 20 to 50°C, and the reaction time is 1 - 48 hr, preferably 2 - 24 hr.

The reactant obtained by the above-mentioned reaction can be appropriately purified by a general purification method, for example, extraction purification, recrystallization, adsorption purification, reprecipitation, column chromatography, ion exchange chromatography and the like.

As specific examples, Examples using a compound having an -S-S- bond and leaving groups such as mesylate group (MsO) and the like at both terminals as a starting material, and involving binding 1-piperazineethanol, and binding liposoluble vitamin or fatty acid are described below (Examples 1-3). Those of ordinary skill in the art can produce the compound of the present invention by appropriately selecting the starting material and performing the reactions according to the method of the Examples in the present specification.

The lipid membrane structure of the present invention is now explained. The lipid membrane structure of the present invention contains a compound represented by the above-mentioned formula (1) as a membrane-constituting lipid. Here, the "lipid membrane structure" in the present invention means a particle having membrane structure wherein the hydrophilic groups of amphipathic lipid are arranged in the interface, facing the aqueous phase side. The "amphiphilic lipid" means a lipid having both a hydrophilic group showing hydrophilicity and a hydrophobic group showing hydrophobicity. Examples of the amphiphilic lipid include cationic lipid, phospholipid and the like.

While the form of the lipid membrane structure of the present invention is not particularly limited, for example, liposome (e.g., unilamellar liposome, multilayer liposome etc.), O/W emulsion, W/O/W emulsion, spherical micelle, worm-like micelle, or unspecified layer structure and the like can be mentioned as a form of dispersion of the cationic lipid of the present invention in an aqueous solvent. The form of the lipid membrane structure of the present invention is preferably a liposome.

The lipid membrane structure of the present invention may further contain, in addition to the cationic lipid of the present invention, other constituent components other than the cationic lipid. Examples of such other constituent component include lipid (phospholipid (phosphatidylinositol, phosphatidylethanolamine, phosphatidylserine, phosphatidic acid, phosphatidylglycerol, phosphatidylcholine etc.), glycolipid, peptide lipid, cholesterol, cationic lipid other than the cationic lipid of the present invention, PEG lipid etc.), surfactant (e.g., 3-[(3-cholamidopropyl)dimethylammonio]propanesulfonate, sodium cholate salt, octylglycoside, N-D-gluco-N-methylalkanamides etc.), polyethylene glycol, protein and the like can be mentioned. The content of other constituent component in the lipid membrane structure of the present invention is generally 5 - 100 mol%, preferably 10 - 90 mol%, more preferably 30 - 70 mol%.

While the content of the cationic lipid of the present invention to be contained in the lipid membrane structure of the present invention is not particularly limited, for example, when the lipid membrane structure is used for the below-mentioned nucleic acid-introducing agent, it contains the cationic lipid of the present invention in an amount sufficient for introducing the nucleic acid. For example, it is generally 5 - 100 mol%, preferably 10 - 90 mol%, more preferably 30 - 70 mol%, of the total lipid amount.

The lipid membrane structure of the present invention can be prepared by dispersing the cationic lipid of the present invention and other constituent components (lipid etc.) in a suitable solvent or dispersing medium, for example, aqueous solvent and alcoholic solvent, and performing an operation to induce organization as necessary.

Examples of the "operation to induce organization" include, but are not limited to, methods known per se such as an ethanol dilution method, a simple hydration method, sonication, heating, vortex, an ether injecting method, a French press method, a cholic acid method, a Ca²⁺ fusion method, a freeze-thaw method, a reversed-phase evaporation method and the like.

A nucleic acid can be introduced into a cell in vivo and/or in vitro by encapsulating the nucleic acid in the lipid membrane structure containing cationic lipid of the present invention and contacting the lipid membrane structure with the cell. Therefore, the present invention provides a nucleic acid-introducing agent, containing the above-mentioned cationic lipid or lipid membrane structure of the present invention.

The nucleic acid-introducing agent of the present invention can introduce any nucleic acid into a cell. Examples of the kind of nucleic acid include, but are not limited to, DNA, RNA, chimera nucleic acid of RNA, DNA/RNA hybrid and the like. While any nucleic acid having 1 to 3 chains can be used, it is preferably a single strand or double strand. The nucleic acid may be other type of nucleotide such as N-glycoside of purine or pyrimidine base or other oligomer having a non-nucleotide backbone (e.g., commercially available peptide nucleic acid (PNA) etc.), other oligomer containing a special bond (said oligomer comprising base pairing or a nucleotide having a configuration permitting attachment of base, which are found in DNA and RNA) and the like. Furthermore, for example, it may be a nucleic acid added with known modification, nucleic acid with a label known in the field, nucleic acid with a cap, methylated nucleic acid, nucleic acid wherein one or more natural nucleotides are substituted by an analog, nucleic acid with intramolecular nucleotidyl modification, nucleic acid with non-charge bond (e.g., methylphosphonate, phosphotriester, phosphoramidate, carbamate and the like), nucleic acid with a charged bond or sulfur-containing bond (e.g., phosphorothioate, phosphorodithioate and the like), nucleic acid with a side chain group such as protein (e.g., nuclease, nuclease inhibitor, toxin, antibody, signal peptide, poly-L-lysine and the like), sugar (e.g., monosaccharide and the like) and the like, nucleic acid with an intercalating compound (e.g., acridine, psoralen and the like), nucleic acid with a chelate compound (e.g., metal, radioactive metal, boron, oxidative metal and the like), nucleic acid containing an alkylating agent, or nucleic acid with a modified bond (e.g., α anomer-type nucleic acid and the like).

The kind of DNA that can be used in the present invention is not particularly limited, and can be selected as appropriate according to the object of use. For example, plasmid DNA, cDNA, antisense DNA, chromosomal DNA, PAC, BAC and the like can be mentioned. Preferred are plasmid DNA, cDNA and antisense DNA, and more preferred is plasmid DNA. A circular DNA such as plasmid DNA and the like can be digested as appropriate with a restriction enzyme and the like, and also used as a linear DNA.

The kind of RNA that can be used in the present invention is not particularly limited, and can be selected as appropriate according to the object of use. For example, siRNA, miRNA, shRNA, antisense RNA, messenger RNA (mRNA), single strand RNA genome, double strand RNA genome, RNA replicon, transfer RNA, ribosomal RNA and the like can be mentioned, with preference given to siRNA, miRNA, shRNA, mRNA, antisense RNA, and RNA replicon.

The nucleic acid used in the present invention is preferably purified by a method generally used by those of ordinary skill in the art.

In one embodiment, the nucleic acid used in the present invention has a low and suppressed CpG sequence frequency, and preferably does not contain a CpG sequence. Using a nucleic acid with a low CpG sequence frequency, the nucleic acid introduced into the cell stays in the cell for a long period, and maintains the physiological effect thereof for a long period. For example, when a plasmid DNA (expression vector) free of a CpG sequence is used as a nucleic acid to be used in the present invention, the object gene can be expressed in a sustained manner for a longer period. In the present specification, the CpG sequence is a 2 base sequence of a type having guanine appearing after cytosine from 5' to 3'. For example, the frequency of the CpG sequence in the nucleic acid used in the present invention is not more than one per 50 bases, preferably not more than one per 100 bases, more preferably not more than one per 1000 bases, most preferably none.

In addition, a CpG sequence induces an innate immune response, and therefore, the development of side effects such as inflammation and the like caused by the innate immune response can be avoided by using a nucleic acid having low and suppressed CpG sequence frequency (preferably, nucleic acid free of CpG sequence) in the present invention. Particularly, since the compound and the lipid membrane structure of the present invention themselves are less stimulatory, and they scarcely induce production of inflammatory cytokine when administered to the body. Thus, the risk of developing side effects such as inflammation and the like caused by innate immune response can be suppressed to the minimum by using the lipid membrane structure of the present invention and a nucleic acid having low and suppressed CpG sequence frequency (preferably, nucleic acid free of CpG sequence) in combination.

The nucleic acid-introducing agent of the present invention may be used in combination with an anti-inflammatory agent, or an anti-inflammatory agent may be encapsulated in a lipid membrane structure. The combined use with an anti-inflammatory agent is a preferable embodiment since it can minimize the risk of developing side effects associated with the introduction of a nucleic acid, as well as further enhance the gene expression efficiency as is also clear from the below-mentioned Examples. Examples of the anti-inflammatory agent include non-steroidal anti-inflammatory agents (e.g., ibuprofen, ketoprofen, naproxen, indomethacin, aspirin, diclofenac, piroxicam, acetaminophen, celecoxib, rofecoxib and the like), and steroidal anti-inflammatory agents (e.g., hydrocortisone, predonisolone, dexamethasone, betamethasone and the like), with preference given to steroidal anti-inflammatory agents. These inflammatory agents may also be used after derivatizing according to the administration form. For example, dexamethasone is preferably fatty acid esterified, particularly preferably used as dexamethasone palmitate. An anti-inflammatory agent can be encapsulated in a lipid membrane structure in the same manner as in the below-mentioned method of encapsulating a nucleic acid in a lipid membrane structure.

The nucleic acid- introducing agent encapsulating a nucleic acid of the present invention can be administered into the body (in vivo) for the purpose of, for example, prophylaxis and/or treatment of a disease. Therefore, the nucleic acid to be used in the present invention preferably has a prophylactic and/or therapeutic activity for a given disease (nucleic acid for prophylaxis or treatment). Examples of such nucleic acid include nucleic acid and the like used for, so-called gene therapy.

To introduce a nucleic acid into cells by the use of the nucleic acid-introducing agent of the present invention, the lipid structure of the present invention encapsulating the nucleic acid is formed by the co-presence of the object nucleic acid when forming the lipid membrane structure of the present invention. For example, when a liposome is formed by an ethanol dilution method, an aqueous nucleic acid solution and a solution of the constituent components (lipid etc.) of the lipid membrane structure of the present invention in an ethanol are vigorously stirred in a vortex and the like, and the mixture is diluted with an appropriate buffer. When a liposome is formed by a simple hydration method, the constituent components (lipid etc.) of the lipid membrane structure of the present invention are dissolved in an appropriate organic solvent, and the solution is placed in a glass container and dried under reduced pressure to evaporate the solvent, whereby a lipid thin film is obtained. Thereto is added an aqueous nucleic acid solution and, after hydration, the mixture is sonicated by a sonicator. The present invention also provides such above-mentioned lipid membrane structure encapsulating a nucleic acid.

As one form of liposome encapsulating a nucleic acid, a multifunctional envelope-type nano device (MEND; hereinafter to be referred to as "MEND") prepared by encapsulating an electrostatic complex of a nucleic acid and a polycation (e.g., protamine) in a liposome can be mentioned (Kogure K et al., Multifunctional envelope-type nano device (MEND) as a non-viral gene delivery system. Adv. Drug Deliv. Rev. 2008). This structure (MEND) can be used as a drug delivery system for selectively delivering a nucleic acid and the like into a particular cell, and useful for, for example, a DNA vaccine, gene therapy of tumor and the like, by introducing antigen gene into dendritic cells.

The particle size of the lipid membrane structure of the present invention encapsulating a nucleic acid is preferably 10 nm - 300 nm, more preferably 100 nm - 200 nm. The particle size can be measured using Zetasizer Nano (Malvern Instruments Ltd.). The particle size of the lipid membrane structure can be appropriately adjusted according to the preparation method of the lipid membrane structure.

The surface charge (zeta potential) of the lipid membrane structure of the present invention encapsulating a nucleic acid is preferably -15 to +10 mV, more preferably -15 to +5 mV. In conventional transgene, particles electrically charged to have a plus surface potential have been mainly used. This is useful as a method for promoting electrostatic interactions with heparin sulfate on the negatively-charged cell surface to enhance uptake into cells. However, positive surface charge may suppress release of nucleic acid from the carrier in the cell due to an interaction with the delivered nucleic acid, or suppress synthesis of protein by an interaction of mRNA and delivered nucleic acid. This problem can be solved by adjusting the surface charge to fall within the above-mentioned. range. The surface charge can be measured using Zetasizer Nano. The surface charge of the lipid membrane structure can be adjusted by the composition of the constituent component of the lipid membrane structure containing the cationic lipid of the present invention.

The lipid membrane structure of the present invention encapsulating the nucleic acid is brought into contact with cells to introduce the encapsulated nucleic acid into the cells. The kind of the cell is not particularly limited, a prokaryotic or eucaryotic cell can be used, with preference given to eucaryote. The kind of the eukaryotic cell is not particularly limited and, for example, vertebrates such as mammals including human (e.g., human, monkey, mouse, rat, hamster, bovine etc.), birds (e.g., chicken, ostrich etc.), amphibia (e.g., frog etc.), fishes (e.g., zebrafish, rice-fish etc.) and the like, invertebrates such as insects (e.g., silk moth, moth, Drosophila etc.) and the like, plants, microorganisms (e.g., yeasts etc.), and the like can be mentioned. More preferably, the target cell in the present invention is an animal or plant cell, more preferably a mammalian cell. The cell may be a culture cell line including a cancer cell, or a cell isolated from an individual or tissue, or a cell of a tissue or tissue piece. The cell may be an adherent cell or a non-adherent cell.

The step of contacting the lipid membrane structure of the present invention encapsulating the nucleic acid with the cell in vitro is specifically explained below.

The cells are suspended in a suitable medium several days before contact with the lipid membrane structure, and cultured under appropriate conditions. At the time of contact with the lipid membrane structure, the cells may or may not be in a proliferative phase.

The culture medium on contact may be a serum-containing medium or a serum-free medium, wherein the serum concentration of the medium is preferably not more than 30 wt%, more preferably not more than 20 wt%, since when the medium contains excess protein such as serum and the like, the contact between the lipid membrane structure and the cell may be inhibited.

The cell density on contact is not particularly limited, and can be appropriately determined in consideration of the kind of the cell and the like. It is generally within the range of 1×10⁴ - 1×10⁷ cells/mL.

For example, a suspension of the aforementioned lipid membrane structure of the present invention encapsulating nucleic acid is added to the thus-prepared cells. The amount of the suspension to be added is not particularly limited, and can be appropriately determined in consideration of the cell number and the like. The concentration of the lipid membrane structure to be contacted with the cells is not particularly limited as long as the desired introduction of the nucleic acid into the cells can be achieved. The lipid concentration is generally 1 - 10 nmol/ml, preferably 10 - 50 nmol/ml, and the concentration of the nucleic acid is generally 0.01 - 100 µg/ml, preferably 0.1 - 10 µg/ml.

After addition of the aforementioned suspension to the cells, the cells are cultivated. The temperature, humidity, CO₂ concentration and the like during the culture are appropriately determined in consideration of the kind of the cell. When the cell is derived from a mammal, temperature about 37°C, humidity about 95% and CO₂ concentration about 5% are generally employed. While the culture period can also be appropriately determined in consideration of the conditions such as the kind of the cell and the like, it is generally 0.1 - 24 hr, preferably 0.2 - 4 hr, more preferably 0.5 - 2 hr. When the above-mentioned culture time is too short, the nucleic acid is not sufficiently introduced into the cells, and when the culture time is too long, the cells may become weak.

By the above-mentioned culture, the nucleic acid is introduced into cells. The culture is further continued preferably by exchanging the medium with a fresh medium, or adding a fresh medium to the medium. When the cell is a mammal-derived cell, the fresh medium preferably contains a serum or nutrition factor.

As mentioned above, a nucleic acid can be introduced into cells not only outside the body (in vitro) but also in the body (in vivo) by using the lipid membrane structure of the present invention. That is, by administration of the lipid membrane structure of the present invention encapsulating the nucleic acid to a subject, the lipid membrane structure reaches and contacts with the target cells, and the nucleic acid encapsulated in the lipid membrane structure is introduced into the cells in vivo. The subject to which the lipid membrane structure can be administered is not particularly limited and, for example, vertebrates such as mammals including human (human, monkey, mouse, rat, hamster, bovine etc.), birds (chicken, ostrich etc.), amphibia (frog etc.), fishes (zebrafish, rice-fish etc.) and the like, invertebrates such as insects (silk moth, moth, Drosophila etc.) and the like, plants and the like can be mentioned. The subject of administration of the lipid membrane structure of the present invention is preferably human or other mammal.

The kind of the target cell is not particularly limited, and a nucleic acid can be introduced into cells in various tissues (e.g., liver, kidney, pancreas, lung, spleen, heart, blood, muscle, bone, brain, stomach, small intestine, large intestine, skin, adipose tissue etc., preferably liver, kidney, pancreas) by using the lipid membrane structure of the present invention.

The cationic lipid and a lipid membrane structure containing same of the present invention show tumor accumulation property and therefore, useful for the treatment of tumor, particularly malignant tumor. Examples of such malignant tumor include, but are not limited to, fibrosarcoma, squamous cell carcinoma, neuroblastoma, breast cancer, gastric cancer, hepatoma, urinary bladder cancer, thyroid gland tumor, urinary epithelial cancer, glia blastoma, acute myeloid leukemia, pancreatic duct cancer, prostate cancer and the like.

The lipid membrane structure of the present invention may be introduced with a compound (e.g., anti-cancer agent and the like) other than a nucleic acid, in addition to the nucleic acid or singly. The method of administering a lipid membrane structure introduced with a compound other than a nucleic acid to a subject (e.g., vertebrate, invertebrate, etc.) is not particularly limited as long as the lipid membrane structure reaches and contacts with the target cells, and the compound to be introduced, which is contained in the lipid membrane structure, can be introduced into the cells, and an administration method known per se (oral administration, parenteral administration (intravenous administration, intramuscular administration, topical administration, transdermal administration, subcutaneous administration, intraperitoneal administration, spray etc.) etc.) can be appropriately selected in consideration of the kind of the compound to be introduced, the kind and the site of the target cell and the like. The dose of the lipid membrane structure is not particularly limited as long as the introduction of the compound into the cells can be achieved, and can be appropriately selected in consideration of the kind of the subject of administration, administration method, the kind of the compound to be introduced, the kind and the site of the target cell and the like.

When the cationic lipid or lipid membrane structure of the present invention is used as a nucleic acid- introducing agent, they can be formulated according to a conventional method.

When the nucleic acid-introducing agent is provide as a reagent for studies, the nucleic acid-introducing agent of the present invention can be provide using the lipid membrane structure of the present invention as it is or a sterile solution or suspension with, for example, water or other physiologically acceptable solution (e.g., aqueous solvent (e.g., malic acid buffer etc.), organic solvent (e.g., ethanol, methanol, DMSO and the like) or a mixture of aqueous solvent and organic solvent etc.). The nucleic acid-introducing agent of the present invention can appropriately contain physiologically acceptable additive known per se (e.g., excipient, vehicle, preservative, stabilizer, binder and the like).

When the nucleic acid-introducing agent is provided as a medicament, the nucleic acid- introducing agent of the present invention can be provided as an oral preparation (e.g., tablet, capsule etc.) or parenteral agent (e.g., injection, spray etc.), preferably parenteral agent (more preferably, injection), by using the lipid membrane structure of the present invention as it is or by blending the lipid membrane structure with a pharmaceutically acceptable known additives such as carrier, flavor, excipient, vehicle, preservative, stabilizer, binder and the like in a unit dosage form required for practicing conventionally admitted preparation formulation.

The nucleic acid- introducing agent of the present invention can also be formulated as a preparation for children as well as for adults.

The nucleic acid-introducing agent of the present invention can also be provided in the form of a kit. The kit can contain, in addition to the cationic lipid or lipid membrane structure of the present invention, a reagent used for the introduction of a nucleic acid. In one embodiment, the nucleic acid-introducing agent (or kit) of the present invention further contains a polycation (e.g., protamine). Using the nucleic acid-introducing agent (or kit) of the present invention in this embodiment, an electrostatic complex of nucleic acid and polycation (e.g., protamine) can be easily encapsulated in the lipid membrane structure of the present invention to constitute MEND, which can be subjected to the intracellular introduction of a nucleic acid.

### [Examples]

The Examples of the present invention are explained in more detail in the following. However, the present invention is not limited in any manner by the Examples.

The abbreviations used for the explanation of Examples each mean as described below.
pDNA: plasmid DNA
Chol: cholesterol
PEG₂₀₀₀-DMG: 1,2-dimyristoyl-sn-glycerol, methoxypolyethylene glycol (PEG MW 2000)
PEG₂₀₀₀-DSG: 1,2-distearoyl-sn-glycerol, methoxypolyethylene glycol (PEG MW 2000)
PEG₅₀₀₀-DSG: 1,2-distearoyl-sn-glycerol, methoxypolyethylene glycol (PEG MW 5000)
DOPE: 1,2-dioleoyl-sn-glycerol-3-phosphoethanolamine
SOPC: 1-stearoyl-2-oleoyl-sn-glycerol-3-phosphocholine
Dex-Pal: dexamethasonepalmitate
DiR: 1,1'-dioctadecyl-3,3,3',3'-tetramethylindocarbocyanine iodide
PBS: phosphate buffered saline
15PGDH: 15-hydroxyprostaglandindehydrogenase

Table 2 shows the name and structure of the cationic lipids produced in the following Examples and Comparative Examples. Comparative Examples 1 and 2 were produced according to Example 1 and Example 5, respectively, of patent document 1.

**Table 2**

| | name of cationic lipid | structure |
|---|---|---|
| Example 1 | TS-PZ4C2 | 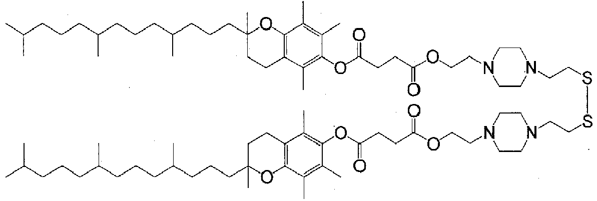 |
| Example 2 | L-PZ4C2 | 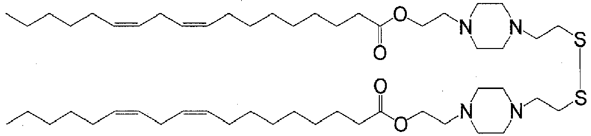 |
| Example 3 | O-PZ4C2 | 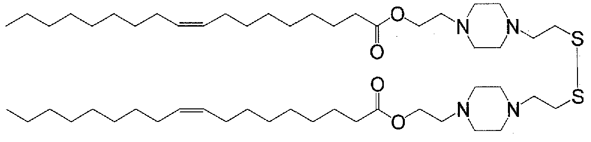 |
| Comparative Example 1 | Myr-C3M | 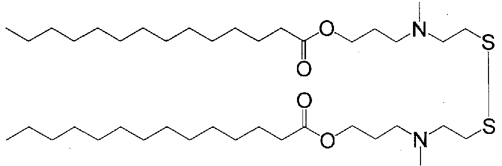 |
| Comparative Example 2 | TS-C3M | 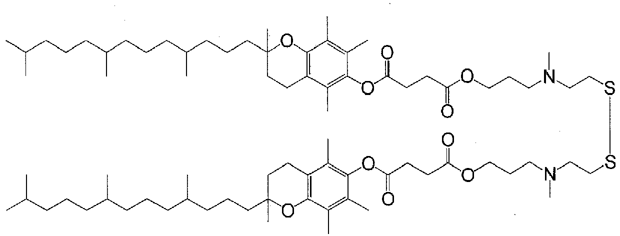 |

### [Example 1] Synthesis of TS-PZ4C2

### <Mesylation>

Acetonitrile (143 mL) was added to bis(2-hydroxyethyl) disulfide (15 g, manufactured by Tokyo Chemical Industry Co., Ltd.) (97 mmol), and the mixture was dissolved at 20 - 25°C. Triethylamine (33.3 g, manufactured by KANTO CHEMICAL CO., INC.) (328 mmol) was added, and the mixture was cooled to 10°C with stirring. Methanesulfonyl chloride (34.5 g, manufactured by KANTO CHEMICAL CO., INC.) (300 mmol) was added dropwise over 1 hr to set the temperature to 20°C or below. After the completion of the dropwise addition, the mixture was reacted at 20 - 25°C for 3 hr. The disappearance of the spot of bis(2-hydroxyethyl) disulfide was confirmed by TLC analysis (eluent: chloroform, iodine color development), and the reaction was completed. Ethanol (29 mL) was added to the reaction solution to discontinue the reaction, and insoluble materials were removed by filtration. 10% Sodium bicarbonate water (150 g) was added to the filtrate, and the mixture was stirred for 5 min and stood for 10 min. The aqueous layer was removed, and the residue was purified by extracting 4 times with sodium bicarbonate water. The obtained organic layer was dehydrated with magnesium sulfate (4.5 g). Insoluble materials were removed by filtration, and the solvent in the filtrate was distilled off by an evaporator to give a brown solid (hereinafter to be referred to as "di-MS form") (29.4 g).

### <¹H-NMR spectrum (600 MHz, CDCl₃)>

The analysis results of ¹H-NMR spectrum of the obtained compound, di-MS form, are shown in the following. δ2.95 - 3.20 ppm(m, CH₃-SO₂ -O-CH₂-CH₂-S-, 10H), δ4.45 - 4.50 ppm(t, CH₃-SO₂-O-CH₂-CH₂-S-, 4H)

### <Tertiary amination>

Acetonitrile (31 mL) was added to di-MS form (1.2 g, 4 mmol), and the mixture was dissolved at 20 - 25°C. Potassium carbonate (1.3 g, manufactured by KANTO CHEMICAL CO., INC.) (10 mmol) was added and the mixture was stirred for 5 min. Thereafter, 4-piperazineethanol (5.0 g, manufactured by Tokyo Chemical Industry Co., Ltd.) (39 mmol) was added and the mixture was reacted at 25 - 35°C for 13 hr. The disappearance of the spot of di-MS form was confirmed by TLC analysis (eluent: chloroform/methanol/28% aqueous ammonia=80/20/2(v/v/v), iodine color development), and the reaction was completed. Insoluble materials were removed by filtration, and the solvent in the filtrate was distilled off by an evaporator. The obtained brown liquid was dissolved in chloroform (25 mL), distilled water (25 mL) was added and the mixture was stirred for 5 min. After stirring, the mixture was stood for 10 min and the aqueous layer was removed. Thereafter, the residue was purified by extracting 2 times with distilled water. The obtained organic layer was dehydrated with magnesium sulfate (0.6 g). Insoluble materials were removed by filtration, and the solvent in the filtrate was distilled off by an evaporator to give a pale-yellow liquid (hereinafter to be referred to as "di-PZ4C2 form") (1.0 g).

### <¹H-NMR spectrum (600 MHz, CDCl₃)>

The analysis results of ¹H-NMR spectrum of the obtained compound, di-PZ4C2 form, are shown in the following. δ2.40 - 2.66 ppm(m, HO-CH₂-CH₂-N-CH₂-CH₂-N-, 20H), δ2.67 - 2.72 ppm(m, -N-CH₂-CH₂-S-, 4H), 2.74 - 2.85 ppm(m, HO-CH₂-, -N-CH₂-CH₂-S-, 6H), 3.60 - 3.65 ppm(t, HO-CH₂-CH₂-, 4H)

### <Acylation>

di-PZ4C2 form (3.0 g, 8 mmol) and D-α-tocopherol succinate (8.4 g, manufactured by SIGMA-ALDRICH) (16 mmol) were dissolved in chloroform (45 mL) at 20 - 25°C. Thereafter, 4-dimethylaminopyridine (0.4 g, manufactured by KOEI CHEMICAL CO., LTD.) (3 mmol) and EDC (4.6 g, manufactured by Tokyo Chemical Industry Co., Ltd.) (24 mmol) were added and the mixture was reacted at 30°C for 4 hr. The disappearance of the spot of D-α-tocopherol succinate was confirmed by TLC analysis (eluent: chloroform/methanol=9/1(v/v), phosphoric acid copper sulfate color development), and the reaction was completed. The reaction solvent was distilled off by an evaporator, and hexane (200 mL) was added. Thereafter, acetonitrile (100 mL) was added, and the mixture was stirred for 5 min. After standing for 10 min, the hexane layer was recovered, and the solvent was distilled off by an evaporator to give a pale-yellow liquid (10.7 g). The liquid (9.0 g) was purified by silica gel column chromatography (eluent: chloroform/methanol=99/1 - 98/2(v/v)) to give the object product TS-PZ4C2 (5.7 g).

### <¹H-NMR spectrum (600 MHz, CDCl₃)>

The analysis results of ¹H-NMR spectrum of the obtained compound, TS-PZ4C2, are shown in the following. δ0.83 - 0.88 ppm(m, (CH₃)₂CH-(CH₂)₃-(CH₃)CH-(CH₂)₃-(CH₃)CH-, 24H), δ1.03 - 1.82 ppm (m, (CH₃)₂CH-(CH₂)₃-(CH₃)CH-(CH₂)₃-(CH₃)CH-(CH₂)₃-(CH₃)C-, -C-CH₂-CH₂-C-C-O-, 52H), δ1.95 - 2.09 ppm(m, Ar-CH₃, 18H), δ2.40 - 2.60 ppm(m, -N-CH₂-CH₂-N-, -C-CH₂-CH₂-C-C-O-, 20H), δ2.61 - 2.68 ppm(m, -O-CH₂-CH₂-N-, -N-CH₂-CH₂-S-, 8H), δ2.75 - 2.84 ppm (m, Ar-O-C(O)-CH₂-, -N-CH₂-CH₂-S-, 8H), δ2.91 - 2.95 ppm (m, Ar-O-C(O)-CH₂-CH₂-, 4H), δ4.21 - 4.25 ppm(t, -C(O)-CH₂-CH₂-N-, 4H)

### [Example 2] Synthesis of L-PZ4C2

### <Acylation>

di-PZ4C2 form (2.5 g, 7 mmol) and linoleic acid (3.7 g, manufactured by NOF CORPORATION) (13 mmol) were dissolved in chloroform (25 mL) at 20 - 25°C. Thereafter, 4-dimethylaminopyridine (0.3 g, 3 mmol) and EDC (3.8 g, 20 mmol) were added and the mixture was reacted at 30°C for 4 hr. The disappearance of the spot of linoleic acid was confirmed by TLC analysis (eluent: chloroform/methanol=9/1(v/v), phosphoric acid copper sulfate color development), and the reaction was completed. The reaction solvent was distilled off by an evaporator, and hexane (57 mL) was added. Thereafter, acetonitrile (24 mL) was added, and the mixture was stirred for 5 min. After standing for 10 min, the hexane layer was recovered, and the solvent was distilled off by an evaporator to give a pale-yellow liquid (4.9 g). The liquid (4.9 g) was purified by silica gel column chromatography (eluent: chloroform/methanol=99/1 - 97/3(v/v)) to give the object product L-PZ4C2 (3.1 g).

### <¹H-NMR spectrum (600 MHz, CDCl₃)>

The analysis results of ¹H-NMR spectrum of the obtained compound, L-PZ4C2, are shown in the following. δ0.87 - 0.91 ppm(t, CH₃-(CH₂)₃-CH₂-, 6H), δ1.25 - 1.38 ppm (m, CH₃-(CH₂)₃-CH₂-, -(CH₂) ₄-CH₂-CH₂-C(O)-, 28H), δ1.58 - 1.63 ppm(m, -(CH₂)₄-CH₂-CH₂-C(O)-, 4H), δ2.00 - 2.07 ppm(m, -CH₂-CH=CH-CH₂-CH=CH-CH₂-, 8H), δ2.30 - 2.32 ppm(t, -(CH₂)₄-CH₂-CH₂-C(O)-, 4H), δ2.50 - 2.70 ppm (m, -N-CH₂-CH₂-N-, -N-CH₂-CH₂-S-, -O-CH₂-CH₂-N-, 24H), δ2.75 - 2.84 ppm (m, -CH=CH-CH₂-CH=CH-, -N-CH₂-CH₂-S-, 8H), δ4.18 - 4.21 ppm(t, -O-CH₂-CH₂-N-, 4H), δ5.30 - 5.41 ppm(m, - CH₂-CH=CH-CH₂-CH=CH-CH₂-, 8H)

### [Example 3] Synthesis of O-PZ4C2

di-PZ4C2 form(0.8 g, 2 mmol) and oleic acid (1.2 g, manufactured by NOF CORPORATION) (4 mmol) were dissolved in chloroform (8 mL) at 20 - 25°C. Thereafter, 4-dimethylaminopyridine (0.1 g, 1 mmol) and EDC (1.2 g, 6 mmol) were added and the mixture was reacted at 30°C for 3 hr. The disappearance of the spot of oleic acid was confirmed by TLC analysis (eluent: chloroform/methanol=9/1(v/v), phosphoric acid copper sulfate color development), and the reaction was completed. The reaction solvent was distilled off by an evaporator, and hexane (12 mL) was added. Thereafter, acetonitrile (5 mL) was added, and the mixture was stirred for 5 min. After standing for 10 min, the hexane layer was recovered, and the solvent was distilled off by an evaporator to give a pale-yellow liquid (1.8 g). The liquid (1.7 g) was purified by silica gel column chromatography (eluent: chloroform/methanol=99/1 - 97/3(v/v)) to give the object product, O-PZ4C2 (1.1 g).

### <¹H-NMR spectrum (600 MHz, CDCl₃)>

The analysis results of ¹H-NMR spectrum of the obtained compound, O-PZ4C2, are shown in the following. δ0.86 - 0.90 ppm (t, CH₃-(CH₂)₆-CH₂-, 6H), δ1.25 - 1.34 ppm(m, CH₃-(CH₂)₆-CH₂-, -CH₂-(CH₂)₄-CH₂-CH₂-C(O)-, 40H), δ1.58 - 1.64 ppm(m, -CH₂-(CH₂)₄-CH₂-CH₂-C(O)-, 4H), δ1.99 - 2.03 ppm(m, -CH₂-CH=CH-CH₂-, 8H), δ2.28 - 2.32 ppm(m, -CH₂- (CH₂) ₄-CH₂-CH₂-C (O)-, 4H), δ2.45 - 2.70 ppm(m, -N-CH₂-CH₂-N-, -O-CH₂-CH₂-N-, -N-CH₂-CH₂-S-, 24H), δ2.80 - 2.85 ppm(m, -N-CH₂-CH₂-S-, 4H), δ4.18 - 4.21 ppm(t, -O-CH₂-CH₂-N-, 4H), δ5.13 - 5.38 ppm(m, -CH₂-CH=CH-CH₂-, 4H)

### [Experimental Example 1]

### 1. Preparation of various MENDs

### Preparation of MEND using Myr-C3M

### (1) Formation of nucleic acid electrostatic complex composed of plasmid DNA (pDNA) and protamine

A solution of pDNA encoding luciferase gene and a protamine (manufactured by CALBIOCHEM) solution were diluted with 10 mM HEPES buffer to 0.15 mg/mL and 96.3 µg/mL, respectively. While stirring 0.15 mg/mL pDNA solution (100 pL), 96.3 µg/mL protamine (100 µL) was added dropwise in small portions to prepare an electrostatic complex of protamine and pDNA (N/P ratio=1.0) as a core of the vector.

### (2) Preparation of MEND by ethanol dilution method

A lipid solution in ethanol was prepared by mixing 5 mM cationic lipid (Myr-C3M), 5 mM phospholipid (SOPC) and 5 mM cholesterol (Chol) at desired ratios to achieve 330 nmol total lipid in an Eppendorf tube, further adding PEG₂₀₀₀-DSG (1 mM ethanol solution) in an amount corresponding to 3 mol% of the total lipid, and adding ethanol to achieve a total volume of 200 µL. While stirring the lipid solution in a vortex mixer, 200 µL of the nucleic acid electrostatic complex (10 mM HEPES; pH 5.3) prepared in [Experimental Example 1] (1) was quickly added, and thereafter 10 mM HEPES buffer (1.6 mL, adjusted to pH 5.3) was added. Furthermore, 10 mM HEPES buffer (2 mL) adjusted to pH5.3 was added to dilute the mixture to an ethanol concentration of 5%. The mixture was concentrated to about 50 µL by ultrafiltration using Amicon Ultra 4 (Millipore) under centrifugation conditions (room temperature, 1000 g, 15 min). Thereafter, it was diluted to 4 mL with 100 mM HEPES buffer (adjusted to pH 7.4), and again concentrated by centrifugation (1000 g, 15 min) under room temperature conditions. Thereafter, it was diluted to 4 mL with 10 mM HEPES buffer (pH 7.4), and again concentrated by centrifugation (1000 g, 15 min) under room temperature conditions. Finally, it was diluted with 10 mM HEPES buffer (pH 7.4) to a desired lipid concentration.

### Preparation of MEND using TS-PZ4C2 or TS-C3M

pDNA solution (1 mg/mL), 100 mM malic acid buffer (pH 4.0), 5 M aqueous sodium chloride solution and sterilized water were mixed, and a solution having final concentrations of 0.1 mg/mL, 20 mM, 40 mM, respectively, was prepared and used as a DNA solution.

A lipid solution in ethanol was prepared by mixing 5 mM cationic lipid (TS-PZ4C2 or TS-C3M), and 10 mM cholesterol (Chol) at desired ratios to achieve 600 nmol total lipid in a 5 mL tube, further adding PEG₂₀₀₀-DMG (5 mM ethanol solution) in an amount corresponding to 3 mol% of the total lipid, and adding ethanol to achieve a total volume of 200 µL. While stirring the lipid solution in a vortex mixer, the above-mentioned DNA solution (300 µL) was quickly added, after which 20 mM malic acid buffer (pH 4.0, containing 100 mM sodium chloride) (500 µL) was added, and phosphate buffered saline was added to dilute the mixture to the ethanol concentration of 10%. Similar operation was repeated three times and phosphate buffered saline was added to an ethanol concentration of 5%. Thereafter, the mixture was concentrated to about 200 µL by ultrafiltration using Amicon Ultra 15 (Millipore, under centrifugation conditions (room temperature, 2267 rpm, 20 min). Thereafter, it was diluted to 15 mL with phosphate buffered saline, and again concentrated by centrifugation (2267 rpm, 20 min) under conditions of room temperature. Finally, it was diluted with phosphate buffered saline to a desired lipid concentration.

### 2. Measurement of particle size, and surface potential of various MENDs

The particle size and the surface potential were measured by the dynamic light scattering method (Zetasizer Nano; Malvern Instruments Ltd.). The particle size and the surface potential of the various MENDs prepared in the above-mentioned 1. are shown in Tables 3 - 5.

**Table 3**

| name of cationic lipid | lipid composition | |
|---|---|---|
| TS-PZ4C2 | cationic lipid:Chol=7:3 | |
| particle size (nm) | PdI | zeta potential (mV) |
| 126.8 | 0.041 | -7.38 |

**[Table 4]**

| name of cationic lipid | lipid composition | |
|---|---|---|
| TS-C3M | cationic lipid:Chol=7:3 | |
| particle size (nm) | PdI | zeta potential (mV) |
| 141.3 | 0.08 | -5.4 |

**[Table 5]**

| name of cationic lipid | lipid composition | |
|---|---|---|
| Myr-C3M | cationic lipid:SOPC:Chol=3:4:3 | |
| particle size (nm) | PdI | zeta potential (mV) |
| 128.3 | 0.188 | 1.88 |

### 3. Results

In any cationic lipid, the electric charge at physiological pH was in a preferable form, -15 - +10 mV.

### [Experimental Example 2] Gene expression activity -1 (gene expression activity in vivo: gene delivery to liver)

### 1. Preparation of various MENDs

Various MENDs were prepared by the method described in [Experimental Example 1].

### 2. Gene expression activity evaluation

The prepared MEND solutions were each administered to 4-week-old male ICR mice from the tail vein in an amount corresponding to 20 µg DNA. The mice were euthanized by a cervical spine destaining method 24, 48 hr later, and the liver was isolated and frozen with liquid nitrogen. They were lysed in a Lysis buffer to prepare homogenates. They were centrifuged at 13,000 rpm, 10 min, 4°C, and the supernatant was collected and used as a measurement sample. The sample solution (20 µL) was mixed with a luciferase substrate (50 µL), and the luciferase activity was measured using Luminescenser-PSN (AB2200 ATTO). In addition, the concentration of the protein in the sample was quantified using a BCA protein assay kit, and the gene expression activity was measured as RLU/mg protein.

### 3. Results

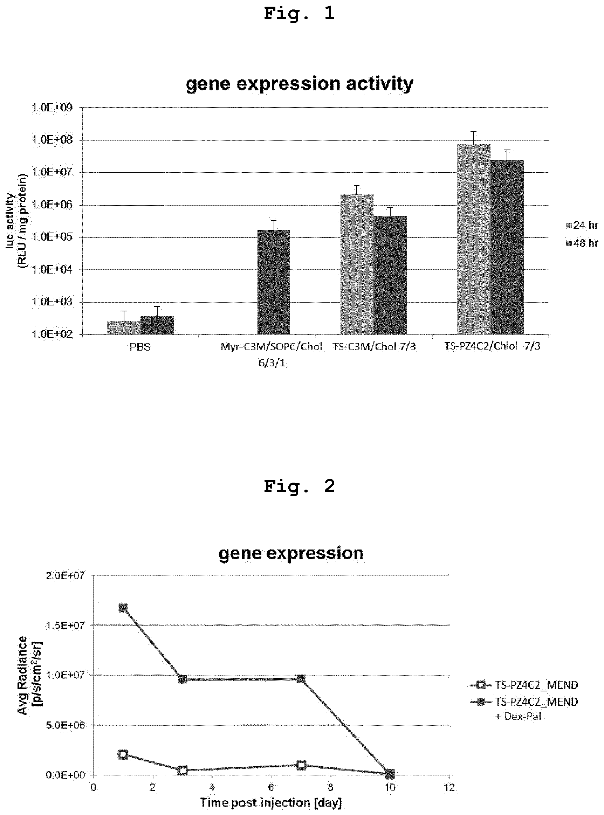

The results are shown in Fig. 1. A higher value, namely, a higher luciferase activity, means a higher gene expression activity. The lipid membrane structure using the cationic lipid of the present invention showed a higher gene expression activity than the lipid membrane structures using the cationic lipids of Comparative Example 1 (patent document 1, Example 1) and Comparative Example 2 (patent document 1, Example 5). It is known that the cationic lipids described in patent document 1 such as TS-C3M, Myr-C3M and the like show high nucleic acid delivery efficiency as compared to cationic lipids such as DOTAP, DODAP and the like (patent document 1). Therefore, it is clear that the cationic lipid of the present invention has gene transfer activity in vivo which is superior to that of DOTAP and DODAP, which are conventional cationic lipids, in addition to TS-C3M and Myr-C3M.

### [Experimental Example 3] Gene expression activity and activity duration in vivo (effect of combined use with anti-inflammatory agent)

### 1. Preparation of MEND

MEND encapsulating dexamethasone palmitate was prepared by adding, during preparation of the MEND described in [Experimental Example 1], an ethanol solution of dexamethasone palmitate to a lipid solution at a final concentration of 0.5 mM.

### 2. Gene expression activity evaluation

The MEND solutions prepared by the method shown in [Experimental Example 3] 1. were each administered to 4-week-old male ICR mice from the tail vein in an amount corresponding to 20 µg DNA. At 1, 3, 7, 10 days from the administration, luciferin (in vivo grade, Promega) corresponding to 3 mg was intraperitoneally administered to the mice, and imaging was performed using IVIS LuminaII (Caliper Life Sciences). An average value of the luminance in the mouse abdomen was calculated as photons/sec/cm²/sr from the obtained images, and used as an index of gene expression activity in the liver.

### 3. Results

The results are shown in Fig. 2. The gene expression activity was improved when an anti-inflammatory agent, dexamethasone palmitate, was encapsulated in a lipid membrane structure using the cationic lipid of the present invention. Furthermore, the gene expression was achieved for 10 days in vivo.

### [Experimental Example 4] Gene expression activity-2 (gene expression activity in vivo: comparison with commercially available nucleic acid-introducing agent)

### 1. Preparation of nucleic acid-introducing agent

Naked-pDNA was diluted with HEPES buffer to 2.4 µg/150 µL. Lipofectamine 2000 (Invitrogen)+pDNA was produced by adding Lipofectamine 2000 to HEPES buffer at 9.6 µL/75 µL, incubating the solution for 5 min at room temperature, mixing same with an equal amount of a solution of pDNA in HEPES buffer at 2.4 µg/75 µL, and incubating the mixture for 20 min at room temperature. TS-PZ4C2_MEND was produced by the ethanol dilution method of [Experimental Example 1] (2) and using PEG₂₀₀₀-DMG as PEG lipid.

### 2. Gene expression activity evaluation

Naked-pDNA and Lipofectamine 2000+pDNA prepared by the method shown in [Experimental Example 4] 1., each in an amount corresponding to 2.4 µg DNA, and TS-PZ4C2_MEND solution in an amount corresponding to 1.2 µg DNA were subcutaneously administered to the back of the neck of 6-week-old female BALB/c mouse. After 24 hr, luciferin (in vivo grade, Promega) corresponding to 3 mg was intraperitoneally administered to the mice, and imaging was performed using IVIS LuminaII (Caliper Life Sciences). The luminance in the neck of the mouse was calculated as photon/sec from the obtained images, and used as an index of gene expression activity.

### 3. Results

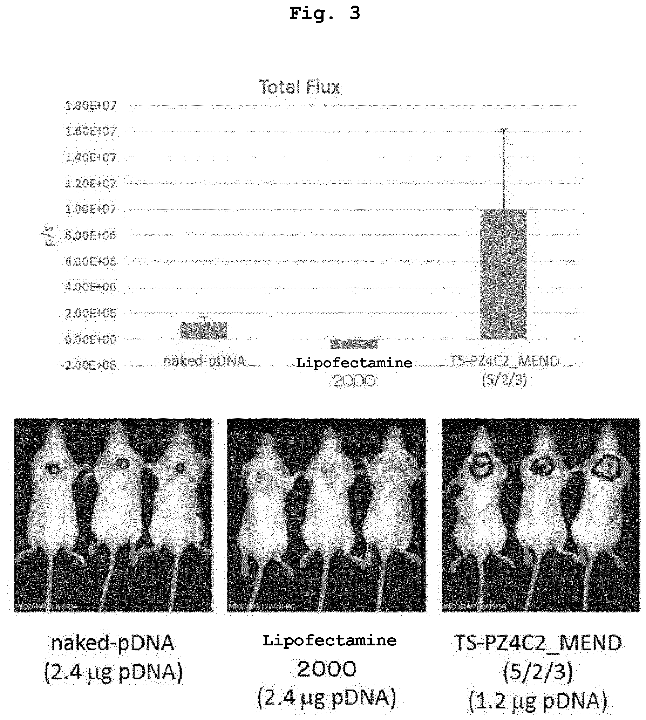

The results are shown in Fig. 3. The activity was higher when a lipid membrane structure using the cationic lipid of the present invention as a nucleic acid-introducing agent than when pDNA only or Lipofectamine 2000, which is a commercially available nucleic acid-introducing agent, was used.

### [Experimental Example 5] Gene expression activity - 3

### 1. Preparation of various MENDs

MENDs having a composition of (cationic lipid:DOPE:Chol)=(5:2:3), (4:3:3), (3:4:3) were produced by the ethanol dilution method of [Experimental Example 1](2) and using DOPE as a phospholipid and PEG₂₀₀₀-DMG as a PEG lipid. MEND having a composition of (cationic lipid:Chol)=(7:3) was prepared by the method described in [Experimental Example 1].

### 2. Gene expression activity evaluation

TS-PZ4C2_MEND solution prepared by the method shown in [Experimental Example 5] 1. and TS-C3M_MEND solution each in an amount corresponding to 1.2 µg DNA were subcutaneously administered to the back of the neck of 6-week-old female BALB/c mouse, and evaluated, by a method similar to that in [Experimental Example 4], 2.

### 3. Results

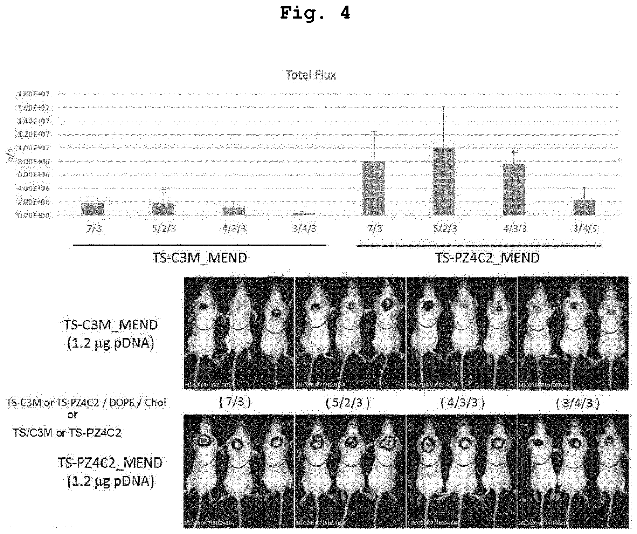

The results are shown in Fig. 4. It is clear that the present invention having two lipid components and four amino groups shows higher gene transfer efficiency than Comparative Example 2 composed of two lipid components and two amino groups.

### [Experimental Example 6]

### 1. Preparation of various MENDs

### Preparation of MEND using TS-PZ4C2 or Myr-C3M

Various MENDs were prepared by the ethanol dilution method in [Experimental Example 1] (2), and DOPC was used as a phospholipid. PEG₅₀₀₀-DSG was used as a PEG lipid, and an amount corresponding to 5 mol% of the total lipid was added in Myr-C3M MEND, and an amount corresponding to 10 mol% was added in TS-PZ4C2 MEND.

### Preparation of MEND using L-PZ4C2 or O-PZ4C2

pDNA solution (1 mg/mL), 100 mM malic acid buffer (pH 4.0), 5 M aqueous sodium chloride solution and sterilized water were mixed, and a solution having a final concentration of 0.1 mg/mL, 20 mM, 40 mM, respectively, was prepared and used as a DNA solution.

A lipid solution in ethanol was prepared by mixing 5 mM cationic lipid (L-PZ4C2, O-PZ4C2), 10 mM cholesterol (Chol), and 10 mM DOPC at desired ratios to achieve 840 nmol total lipid in a 5 mL tube, further adding PEG₅₀₀₀-DSG (1 mM ethanol solution) in an amount corresponding to 5 mol% of the total lipid, and adding ethanol to achieve a total amount of 200 µL (80% ethanol solution) and 20 mM malic acid buffer (pH 4.0). While stirring the lipid solution in a vortex mixer, the above-mentioned DNA solution (200 µL) was quickly added, after which 20 mM malic acid buffer (pH 4.0) (1600 µL) was added, phosphate buffered saline was added, and the mixture was diluted to an ethanol concentration of 8%. Similar operation was repeated three times and phosphate buffered saline was added to an ethanol concentration of 4%. Thereafter, using Amicon Ultra 15 (Millipore), the mixture was concentrated to about 200 µL by ultrafiltration under centrifugation condition (room temperature, 2267 rpm, 30 min). Thereafter, the mixture was diluted with phosphate buffered saline to 15 mL, and concentrated again by centrifugation (2267 rpm, for 30 min) under room temperature conditions. Finally, it was diluted to a desired lipid concentration with phosphate buffered saline.

### 2. Measurement of particle size and surface potential of various MENDs

The particle size and the surface potential were measured by the dynamic light scattering method (Zetasizer Nano). The particle size and the surface potential of the various MENDs prepared by in the above-mentioned 1. are shown in Tables 6 - 9.

**[Table 6]**

| name of cationic lipid | lipid composition | |
|---|---|---|
| Myr-C3M | cationic lipid:DOPC:Chol=3:4:3 | |
| particle size (nm) | PdI | zeta potential (mV) |
| 113.9 | 0.134 | -0.135 |

**[Table 7]**

| name of cationic lipid | lipid composition | |
|---|---|---|
| TS-PZ4C2 | cationic lipid:Chol=7:3 | |
| particle size (nm) | PdI | zeta potential (mV) |
| 104.8 | 0.121 | -0.897 |

**[Table 8]**

| name of cationic lipid | lipid composition | |
|---|---|---|
| L-PZ4C2 | cationic lipid:DOPC:Chol=6:1:3 | |
| particle size (nm) | PdI | zeta potential (mV) |
| 108.7 | 0.094 | 0.144 |

**[Table 9]**

| name of cationic lipid | lipid composition | |
|---|---|---|
| O-PZ4C2 | cationic lipid:DOPC:Chol=6:1:3 | |
| particle size (nm) | PdI | zeta potential (mV) |
| 115.2 | 0.083 | -0.294 |

### 3. Results

In any cationic lipid, the electric charge at physiological pH was in a preferable form, -15 - +10 mV.

### [Experimental Example 7] Evaluation of organ accumulation property in vivo

### 1. Preparation of various MENDs

Fluorescence-labeled MEND was prepared by adding, during preparation of the MEND described in [Experimental Example 6], an ethanol solution of DiR to a lipid solution at a final concentration of 3.6 µM.

### 2. Evaluation of accumulation property in each organ

Cancer carrying mice were produced by subcutaneously administering a suspension of mouse breast cancer-derived cancer cell line 4T1 cells (1x10⁶ cells/mouse) to 6-week-old female BALB/c mice. After 7 days from the subcutaneous transplantation of the cancer cells, the prepared MEND solutions were each administered from the tail vein in an amount corresponding to DiR 1 nmol. The mice were euthanized by a cervical spine destaining method 24 hr later, and each organ was isolated, and imaging was performed using IVIS LuminaII (Caliper Life Sciences) (excitation wavelength: 710 nm, detection: ICG filter). The luminance of each of the organ, liver and tumor was calculated as [photons/sec]/[µW/cm²] from the obtained images, and used as an index of organ accumulation property, liver accumulation property, and tumor accumulation property.

### 3. Results

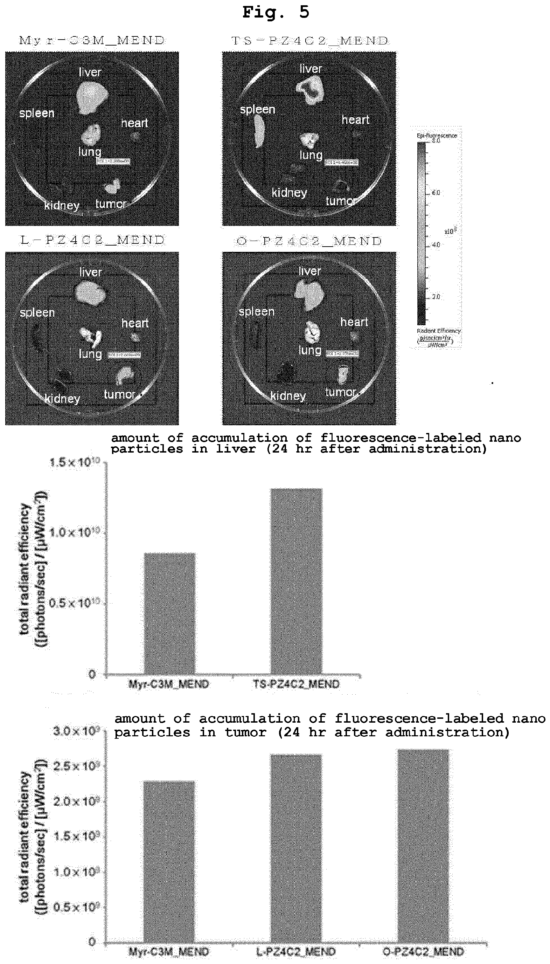

The results are shown in Fig. 5. The lipid membrane structure using the cationic lipid TS-PZ4C2 of the present invention showed a higher accumulation in the liver than the lipid membrane structure using the cationic lipid of Comparative Example 1 (patent document 1, Example 1). In addition, the lipid membrane structure using the cationic lipid L-PZ4C2 or O-PZ4C2 of the present invention showed a higher accumulation in the tumor than the lipid membrane structure using the cationic lipid of Comparative Example 1 (patent document 1, Example 1).

### [Experimental Example 8] Gene expression activity (gene deliver into tumor) in vivo

### 1. Preparation of various MENDs

Various MENDs were prepared according to the method described in [Experimental Example 6].

### 2. Gene expression activity evaluation

Cancer-carrying mice were produced by subcutaneous administration of a suspension of mouse breast cancer-derived cancer cell line 4T1 cells (1x10⁶ cells/mouse) to 6-week-old female BALB/c mice. At 7 days after the subcutaneous transplantation of the cancer cells, the prepared MEND solutions were administered each in an amount corresponding to 25 mg DNA from the tail vein. The mice were euthanized by a cervical spine destaining method at 48 hr after the administration, the tumor was isolated and frozen with liquid nitrogen. The tumor was lysed in a Lysis buffer to prepare homogenate. The homogenate was centrifuged at 13,000 rpm, 10 min, 4°C, and the supernatant was collected and used as a measurement sample. The sample solution (20 µL) was mixed with a luciferase substrate (50 µL), and the luciferase activity was measured using Luminescenser-PSN (AB2200 ATTO). In addition, the concentration of the protein in the sample was quantified using a BCA protein assay kit, and the gene expression activity was measured as RLU/mg protein.

### 3. Results

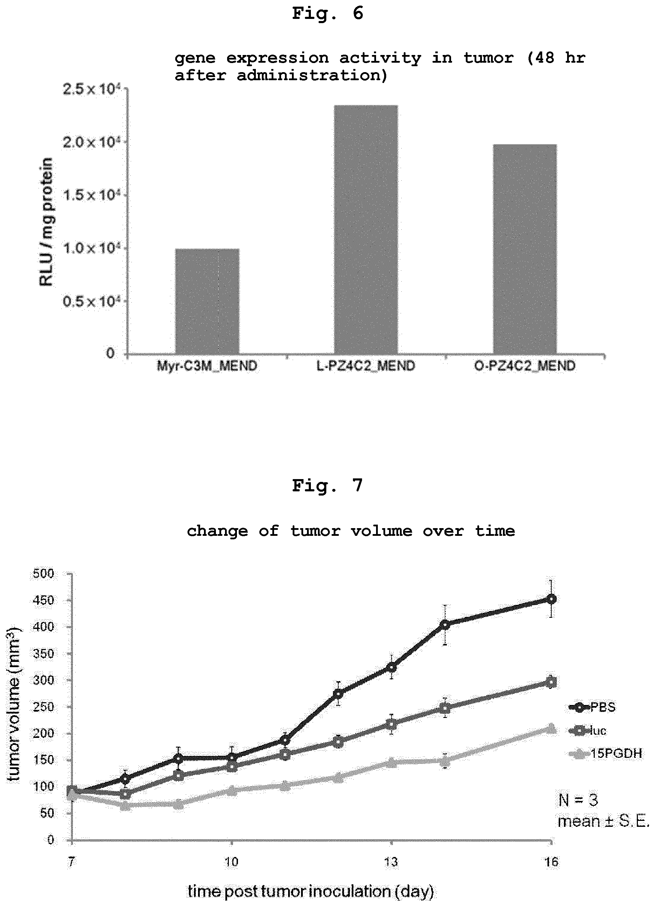

The results are shown in Fig. 6. A higher value, namely, a higher luciferase activity, means a higher gene expression activity. The lipid membrane structure using the cationic lipid (L-PZ4C2 or O-PZ4C2) of the present invention showed a higher gene delivery expression activity to tumor than the lipid membrane structures using the cationic lipids of Comparative Example 1 (patent document 1, Example 1). It is known that Myr-C3M shows higher nucleic acid delivery efficiency as compared to conventional cationic lipids such as DOTAP, DODAP and the like. Therefore, it is suggested that L-PZ4C2 and O-PZ4C2 of the present invention has genet transfer activity into tumor which is superior to that of conventional cationic lipids, in addition to Myr-C3M.

### [Experimental Example 9] Antitumor effect in gene delivery using MEND

### 1. Preparation of plasmid DNA (pDNA) solution

As the DNA to be carried, pDNA encoding luciferase gene or 15PGDH gene was used.

### 2. Preparation of MEND

MEND was produced by the method of [Experimental Example 6] and using L-PZ4C2 as the cationic lipid.

### 3. Evaluation of antitumor effect

Cancer carrying mice were produced by subcutaneously administering a suspension of mouse breast cancer-derived cancer cell line 4T1 cells (1x10⁶ cells/mouse) to 6-week-old female BALB/c mice. From 7 days after the subcutaneous transplantation of the cancer cells, the MEND solution prepared in the above-mentioned 2. was administered each in an amount corresponding to 30 mg DNA from the tail vein once every 3 days, 3 times in total. The minor axis and the major axis of the tumor were measured over time, and the volume was calculated as volume (mm³) = minor axis (mm)² x major axis (mm) x 0.52.

### 4. Results

The results are shown in Fig. 7. A lower value means that enlargement of tumor is suppressed and the antitumor effect is high. It was found that MEND using the cationic lipid of the present invention encapsulating a gene significantly suppresses enlargement of tumor.

### [Industrial Applicability]

According to the present invention, since nucleic acid can be intracellularly introduced with high efficiency, it is useful for gene therapy and biochemical experiments.

This application is based on a patent application No. 2015-16786 filed in Japan (filing date: January 30, 2015).

## Claims

1. A cationic lipid represented by the formula (1) 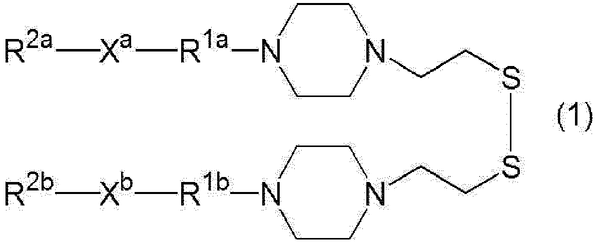
in the formula (1), R^{1a} and R^{1b} are each independently an alkylene group or oxydialkylene group having not more than 8 carbon atoms,
X^{a} and X^{b} are each independently an ester bond, an amide bond, a carbamate bond, or an ether bond, and
R^{2a} and R^{2b} are each independently a sterol residue, a liposoluble vitamin residue, or an aliphatic hydrocarbon group having 13 - 23 carbon atoms.

2. The cationic lipid according to claim 1, wherein R^{1a} and R^{1b} are each independently an alkylene group.

3. The cationic lipid according to claim 1 or 2, wherein X^{a} and X^{b} are ester bonds.

4. The cationic lipid according to any one of claims 1 to 3, wherein R^{2a} and R^{2b} are each independently a liposoluble vitamin residue, or an aliphatic hydrocarbon group having 13 - 23 carbon atoms.

5. The cationic lipid according to any one of claims 1 to 4, wherein R^{2a} and R^{2b} are each independently a liposoluble vitamin residue.

6. The cationic lipid according to any one of claims 1 to 4, wherein R^{2a} and R^{2b} are each independently an aliphatic hydrocarbon group having 13 - 23 carbon atoms.

7. A lipid membrane structure comprising the cationic lipid according to any one of claims 1 to 6 as a membrane-constituting lipid.

8. A nucleic acid-introducing agent, which comprises the cationic lipid according to any one of claims 1 to 6 or the lipid membrane structure according to claim 7.

9. A nucleic acid-introducing agent, which is the cationic lipid according to any one of claims 1 to 6, or the lipid membrane structure according to claim 7, encapsulating an anti-inflammatory agent.

10. A method of delivering a nucleic acid into a cell, comprising contacting the nucleic acid-introducing agent according to claim 8 or 9, which encapsulates the nucleic acid, with the cell in vitro.

11. A nucleic acid-introducing agent according to claim 8 or 9, which encapsulates the nucleic acid, for use in a method of therapy which comprises introducing the nucleic acid into a target cell of a living organism.

## Patentansprüche

1. Kationisches Lipid, dargestellt durch die Formel (1): 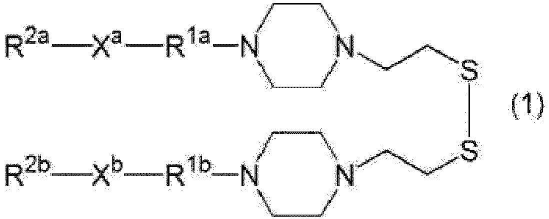
wobei in der Formel (1) R^{1a} und R^{1b} jeweils unabhängig eine Alkylengruppe oder Oxydialkylengruppe mit nicht mehr als 8 Kohlenstoffatomen sind;
X^{a} und X^{b} jeweils unabhängig eine Esterbindung, eine Amidbindung, eine Carbamatbindung oder eine Etherbindung sind; und
R^{2a} und R^{2b} jeweils unabhängig ein Sterolrest, ein Rest eines fettlöslichen Vitamins oder eine aliphatische Kohlenwasserstoffgruppe mit 13 bis 23 Kohlenstoffatomen sind.

2. Kationisches Lipid gemäß Anspruch 1, wobei R^{1a} und R^{1b} jeweils unabhängig eine Alkylengruppe sind.

3. Kationisches Lipid gemäß Anspruch 1 oder 2, wobei X^{a} und X^{b} Esterbindungen sind.

4. Kationisches Lipid gemäß einem der Ansprüche 1 bis 3, wobei R^{2a} und R^{2b} jeweils unabhängig ein Rest eines fettlöslichen Vitamins oder eine aliphatische Kohlenwasserstoffgruppe mit 13 bis 23 Kohlenstoffatomen sind.

5. Kationisches Lipid gemäß einem der Ansprüche 1 bis 4, wobei R^{2a} und R^{2b} jeweils unabhängig ein Rest eines fettlöslichen Vitamins sind.

6. Kationisches Lipid gemäß einem der Ansprüche 1 bis 4, wobei R^{2a} und R^{2b} jeweils unabhängig eine aliphatische Kohlenwasserstoffgruppe mit 13 bis 23 Kohlenstoffatomen sind.

7. Lipidmembranstruktur, die das kationische Lipid gemäß einem der Ansprüche 1 bis 6 als membranbildendes Lipid umfasst.

8. Nucleinsäureeinführungsmittel, das das kationische Lipid gemäß einem der Ansprüche 1 bis 6 oder die Lipidmembranstruktur gemäß Anspruch 7 umfasst.

9. Nucleinsäureeinführungsmittel, das das kationische Lipid gemäß einem der Ansprüche 1 bis 6 oder die Lipidmembranstruktur gemäß Anspruch 7, in dem oder der ein entzündungshemmendes Mittel eingekapselt ist, ist.

10. Verfahren zur Abgabe einer Nucleinsäure in eine Zelle, umfassend das In-Kontakt-Bringen des Nucleinsäureeinführungsmittels gemäß Anspruch 8 oder 9, in dem die Nucleinsäure eingekapselt ist, mit der Zelle in vitro.

11. Nucleinsäureeinführungsmittel gemäß Anspruch 8 oder 9, in dem die Nucleinsäure eingekapselt ist, zur Verwendung in einem Therapieverfahren, das das Einführen der Nucleinsäure in eine Zielzelle eines lebenden Organismus umfasst.

## Revendications

1. Lipide cationique représenté par la formule (1) : 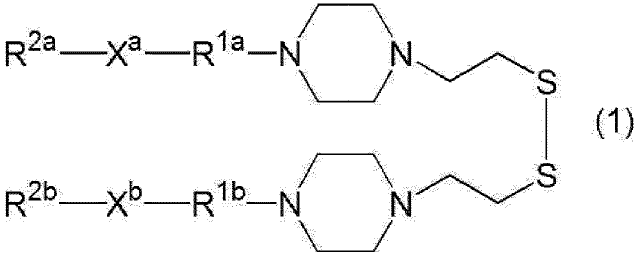
dans la formule (1), R^{1a} et R^{1b} sont chacun indépendamment un groupe alkylène ou un groupe oxydialkylène n'ayant pas plus de 8 atomes de carbone,
X^{a} et X^{b} sont chacun indépendamment une liaison ester, une liaison amide, une liaison carbamate ou une liaison éther, et
R^{2a} et R^{2b} sont chacun indépendamment un résidu de stérol, un résidu de vitamine liposoluble ou un groupe hydrocarboné aliphatique ayant 13 à 23 atomes de carbone.

2. Lipide cationique selon la revendication 1, dans lequel R^{1a} et R^{1b} sont chacun indépendamment un groupe alkylène.

3. Lipide cationique selon la revendication 1 ou 2, dans lequel X^{a} et X^{b} sont des liaisons ester.

4. Lipide cationique selon l'une quelconque des revendications 1 à 3, dans lequel R^{2a} et R^{2b} sont chacun indépendamment un résidu de vitamine liposoluble ou un groupe hydrocarboné aliphatique ayant 13 à 23 atomes de carbone.

5. Lipide cationique selon l'une quelconque des revendications 1 à 4, dans lequel R^{2a} et R^{2b} sont chacun indépendamment un résidu de vitamine liposoluble.

6. Lipide cationique selon l'une quelconque des revendications 1 à 4, dans lequel R^{2a} et R^{2b} sont chacun indépendamment un groupe hydrocarboné aliphatique ayant 13 à 23 atomes de carbone.

7. Structure de membrane lipidique comprenant le lipide cationique selon l'une quelconque des revendications 1 à 6 en tant que lipide constitutif des membranes.

8. Agent d'introduction d'acide nucléique, qui comprend le lipide cationique selon l'une quelconque des revendications 1 à 6 ou la structure de membrane lipidique selon la revendication 7.

9. Agent d'introduction d'acide nucléique, qui est le lipide cationique selon l'une quelconque des revendications 1 à 6, ou la structure de membrane lipidique selon la revendication 7, encapsulant un agent anti-inflammatoire.

10. Procédé d'administration d'un acide nucléique dans une cellule, comprenant la mise en contact de l'agent d'introduction d'acide nucléique selon la revendication 8 ou 9, qui encapsule l'acide nucléique, avec la cellule *in vitro.*

11. Agent d'introduction d'acide nucléique selon la revendication 8 ou 9, qui encapsule l'acide nucléique, destiné à être utilisé dans un procédé thérapeutique qui comprend l'introduction de l'acide nucléique dans une cellule cible d'un organisme vivant.
